# EUROPÄISCHE PATENTSCHRIFT

(11) **EP 1 674 049 B1**
(45) Veröffentlichungstag und Bekanntmachung des Hinweises auf die Patenterteilung: **24.06.2009**
(21) Anmeldenummer: 05023761.9
(22) Anmeldetag: 31.10.2005
(51) Int. Cl.: A61F 2/14

(54) **Künstlicher in ein Auge implantierbarer Irisersatz**
Artificial iris implant for an eye
Iris artificiel implantable dans l'oeil

(30) Priorität: 22.12.2004 DE 102004061943
(43) Veröffentlichungstag der Anmeldung: 28.06.2006
(73) Patentinhaber: *Acri. Tec GmbH, 16761 Hennigsdorf (DE)
(72) Erfinder: Hermeking, Heino, 42283 Wuppertal (DE); Serester, Alexander, 93077 Bad Abbach (DE)
(74) Vertreter: Nöth, Heinz

(56) Entgegenhaltungen:
- EP-A- 0 319 154
- DE-A1- 3 926 536
- DE-A1- 10 320 584
- DE-A1- 19 850 807
- FR-A- 2 696 340
- US-A- 5 108 427
- US-A- 5 628 797
- US-B1- 6 280 469

## Beschreibung

Die Erfindung betrifft einen künstlichen in ein Auge implantierbaren Irisersatz nach dem Oberbegriff des Patentanspruches 1. Ein derartiger aus DE 198 50 807 A1 bekannter Irisersatz beinhaltet einen mehrlagigen aus bioverträglichen Folien gebildeten Prothesekörper mit einer mittleren Apertur. Die mittlere Apertur wird von phototrop ausgebildeten, konzentrisch angeordneten ringförmigen Elementen umfasst. Ein diesen inneren Ringbereich umfassender äußerer Ringbereich ist im Wesentlichen lichtundurchlässig ausgebildet. Ferner besitzt der bekannte Irisersatz eine pheriphäre Haptik zur Positionierung im Auge. Ein derartiger Irisersatz ist ferner aus US-A 5,108,427 bekannt.

Ferner ist aus DE 103 20 584 A1 ein Irisersatz bekannt, dessen Prothesekörper aus um Faltlinien faltbaren Segmenten besteht. Die Segmente werden mit elastischen Klebefalzen an den Faltlinien zusammen gehalten, wobei Rundlöcher im Prothesekörper ein Durchdringen der Segmente mit dem elastischen Material der Klebefalze ermöglichen.

Aufgabe der Erfindung ist es, einen Irisersatz der eingangsgenannten Art zu schaffen, an welchem an jeweilige Implantationsorte im Auge angepasste Haptiken in einfacher Weise befestigt werden können.

Diese Aufgabe wird erfindungsgemäß durch die Merkmale des Patentanspruches 1 gelöst. Vorteilhafte Weiterbildungen der Erfindung sind in den Unteransprüchen angegeben.

Bei der Erfindung besitzt der Prothesekörper einen inneren die Apertur umfassenden Ringbereich, welcher phototrop ausgebildet ist. Um den inneren phototropen Ringbereich ist ein äußerer Ringbereich des Prothesekörpers vorgesehen, welcher im wesentlichen lichtundurchlässig ausgebildet ist, d.h. einen Transmissionsgrad von geringer als etwa 3% und gegen Null gehend hat. Der Prothesekörper ist als kreisrunde Scheibe ausgebildet und kann im Kapselsack des Auges implantiert sein, wobei sich der Außendurchmesser des Prothesekörpers etwa dem Äquator des Kapselsackes anpasst. Durch Einfärben, Drucken oder ähnlich geeignetes Verfahren kann der äußere Ringbereich mit einem der natürlichen Augeniris nachgebildeten Muster versehen sein.

Vorzugsweise besteht der äußere Ringbereich aus einem weichen Material, beispielsweise Weichacrylaten, HEMA, flexiblem Silikonkautschuk und dergleichen.

Zur Positionierung ist am äußeren Ringbereich ferner eine Haptik vorgesehen sein, deren Aufbau und Ausbildung an den jeweiligen Implantationsort im Auge angepasst ist. Neben einer endokapsulären Lokalisation ist auch eine Sulcuspositionierung oder die Enklavation im Irisrestgewebe möglich. Auch eine transsklerare Fixaktion des Irisprothesekörpers ist möglich. Für eine endokapsuläre Lokalisation kann im äußeren Ringbereich ein den phototropen inneren Ringbereich umgebender Stabilisierungsring aus einem härteren Material als das Material des äußeren Ringbereiches eingelagert sein. Der Stabilisierungsring kann beispielsweise aus Polymethylmethacrylat (PMMA) oder Polycarbonat bestehen. Hierzu kann im äußeren Ringbereich ein Hohlraum vorgesehen sein, in welchem der beispielsweise nach Art eines Spannringes ausgebildete Stabilisierungsring eingelagert wird.

Für die Sulcuspositionierung kann ein Träger (Haptik) vorgesehen sein, welcher in seinem Abstützbereich an den Sulcus angepasst ist. Hierzu kann der Träger kreisbogenförmige Stützhaptiken aufweisen, die am Sulcus positioniert werden und über radial verlaufende Stützen des Trägers den Prothesenkörper zentrisch zwischen den kreisbogenförmigen Stützhaptiken positionieren. Es können vorzugsweise zwei kreisbogenförmige Stützhaptiken vorgesehen sein, die diametral zueinander am Träger vorgesehen sind.

Zur Irisenklavation können am Träger Fixationsklammern, beispielsweise in Zangenform vorgesehen sein, mit welchen der Prothesekörper am Irisgewebe fixiert werden kann. Vorzugsweise sind in gleichen Winkelabständen voneinander drei Fixationsringe vorgesehen.

Für eine Sklerafixation kann der Träger Fixierungsösen aufweisen. Vorzugsweise werden drei in gleichen Winkelabständen voneinander angeordnete Fixationsösen vorgesehen. Mit Hilfe von durch die Fixationsösen geführten Fäden erfolgt die Befestigung an der Augensklera.

Der Prothesekörper besitzt im äußeren Ringbereich Befestigungsstellen, beispielsweise in Form von Öffnungen, in denen die jeweilige Haptik bzw. das jeweilige Trägersystem am Prothesekörper, beispielsweise durch Einstecken befestigt werden kann. Für die verschiedenen Fixationsformen, welche oben beschrieben wurden, kann ein einheitlich gestalteter Prothesekörper vorgesehen sein, an welchem beispielsweise durch Einklipsen oder Aufstecken das geeignete Trägersystem, beispielsweise eine Haptik, zu befestigen ist. Somit ist der Prothesekörper ubiquitär, insbesondere im Vorderabschnitt des Auges einsetzbar. An jedem Prothesekörper können hierzu im äußeren Ringbereich zwei Öffnungen vorgesehen sein, in die Steckköpfe, welche am jeweiligen Trägersystem vorgesehen sind, mit Schnappverschluss aufgenommen werden.

Derartige Fixationsklammern sind aus European Journal Ref. Surgery, Vol. 1, Seiten 41 bis 43, Arch 1989 sowie aus US 4,215,440 zur Befestigung von Intraokularlinsen bekannt. Zur Fixierung wird Irisgewebe zwischen die Klammern eingeklemmt.

Durch die phototrope Ausbildung des inneren Ringbereiches des Prothesekörpers erreicht man in diesem Ringbereich eine von der Belichtung abhängige Lichttransmission. Bei wenig Licht, d.h. bei Dunkelheit ist der innere Ringbereich transparent. Die Lichtdurchlässigkeit wird bei Belichtung reduziert. Bei starker Belichtung wird das Material des inneren Ringbereiches dunkel, d.h. annähernd lichtundurchlässig. Man erhält eine von der Belichtung abhängige Lichtdurchlässigkeit. Auf diese Weise erreicht man eine angenäherte Anpassung der Funktion des künstliches Irisersatzes an die Funktion der natürlichen Iris.

In der Apertur kann vorzugsweise in Form einer Steckprothese ein optisches Teil, insbesondere eine Linse, vorzugsweise Plus-brechende Linse angeordnet sein.

Es ist auch möglich, den als Steckprothese ausgebildeten optischen Teil in einem ringförmigen Bereich mit Innendurchmesser von etwa 3,0 mm und Außendurchmesser von etwa 5,0 mm bis 6,0 mm phototrop zu gestalten. Ein äußerer Ringbereich des optischen Teils ist annährend lichtundurchlässig. Dieser Bereich kann einen Außendurchmesser von ca. 7,0 mm aufweisen.

Ferner ist es möglich, den Prothesekörper als Intraokularlinse auszubilden und einen inneren phototropen Ringbereich mit einem Innendurchmesser von ca. 3,0 mm und einen Außendurchmesser von etwa 5,0 bis 6,0 mm an der Intraokularlinse vorzusehen. Die Apertur (lichtdurchlässiger Bereich) im Zentrum der Intraokularlinse kann auch einen Durchmesser von bis zu 4,0 mm aufweisen. Der äußere Ringbereich der Intraokularlinse mit einem Innendurchmesser von 5,0 bis 6,0 mm bis wenigstens 9,0 mm oder darüber hinaus bis etwa 11,0 mm ist im wesentlichen lichtundurchlässig ausgebildet.

Anhand der Figuren wird an Ausführungsbeispielen die Erfindung noch näher erläutert.

Es zeigt
- Fig. 1: in Draufsicht einen kreisringförmigen Prothesekörper, welcher ein Ausführungsbeispiel für einen künstlichen in ein Auge implantierbaren Irisersatz ist;
- Fig. 2: eine schnittbildliche Darstellung des Prothesekörpers der Fig. 1;
- Fig. 3: den Prothesekörper der Fig. 1 und 2 mit aufgestecktem Trägersystem (Haptik) in Draufsicht;
- Fig. 4: eine Seitenansicht des Trägersystems der Fig. 3;
- Fig. 5: eine Draufsicht des in den Fig. 1 und 2 dargestellten Prothesekörpers mit einem weiteren Trägersystem für Irisenklavation;
- Fig. 6: in Draufsicht ein Prothesekörper der Fig. 1 und 2 mit einem Trägersystem für Sklerafixation;
- Fig. 7: einen Prothesekörper, welcher nach Art der Fig. 1 und 2 ausgebildet sein kann, für eine endokapsuläre Positionierung; und
- Fig. 8: ein als Steckoptik ausgebildetes optisches Bauelement, welches in der Apertur des Prothesekörpers der Fig. 1 und 2 oder in den Aperturen der in den Ausführungsbeispielen der Fig. 3 bis 7 gezeigten Prothesekörper angeordnet werden kann.

Ein ringförmiger Prothesekörper 1 besteht aus augenverträglichem Material. Der Prothesekörper 1 weist eine mittlere kreisrunde Apertur 4 auf. Die Apertur 4 wird von einem inneren Ringbereich 2 des Prothesekörpers 1 umfasst. Dieser innere Ringbereich ist mittels einer oder mehreren eingelagerter oder eingefärbter photochromer Substanzen phototrop ausgebildet. Der innere Ringbereich 2 wird von einem äußeren Ringbereich 3 umfasst. Der äußere Ringbereich 3 ist im wesentlichen lichtundurchlässig ausgebildet, d.h. er besitzt einen Transmissionsgrad von geringer als etwa 3%. Der äußere Ringbereich 3 kann eine der natürlichen Iris nachgebildete Einfärbung, insbesondere durch Druck erzeugte Nachbildung der natürlichen Iris aufweisen. Ferner kann zur Bildung der Lichtundurchlässigkeit eine opake Schicht am äußeren Ringbereich 3 vorgesehen sein.

Der Durchmesser der Apertur 4 beträgt mindestens 3,0 mm und kann beispielsweise bis etwa 4,0 mm betragen. Der Außendurchmesser des inneren Ringbereiches 2 beträgt etwa 5,0 bis 8,0 mm. Der Außendurchmesser des äußeren Ringbereiches 3 beträgt etwa 9,0 bis 13,0 mm.

Der innere Ringbereich 2 kann als Ring aus dem phototropen Material bestehen und in den Innenumfang des äußeren Ringes 3 mit arretierender Wirkung zwischen dem Außenumfang des inneren Ringbereiches 2 und dem Innenumfang des äußeren Ringbereiches 3 eingesetzt sein. Hierzu kann der Außenumfang des phototropen Ringes mit einer umlaufenden konkaven Nut ausgestattet sein, in die der entsprechend konvex ausgebildete Innenumfang des äußeren als Ring ausgebildeten Ringbereiches gegebenenfalls mit Presssitz eingeschnappt ist. Der konvex/konkav ausgebildete Formschluss zwischen den Ringen kann auch umgekehrt an den Ringen angeordnet sein. Ferner ist es möglich, den inneren Ringbereich 2 mit wenigstens einer photochromen Sustanz einzufärben oder eine derartige Substanz in das Material einzulagern. Derartige Substanzen sind beispielsweise aus US 4,286,957 und US 4,342,668 bekannt. Bekannt sind ferner spiro(indolin)-artige photochrome Verbindungen, wie Spiro(indolin)-pyridobenzoxazine, spiro(indolin)-naphthoxazine, Spiro(indolin)-naphthopyrane und Spiro(indolin)chinopyrane (DE 36 29 624 C2).

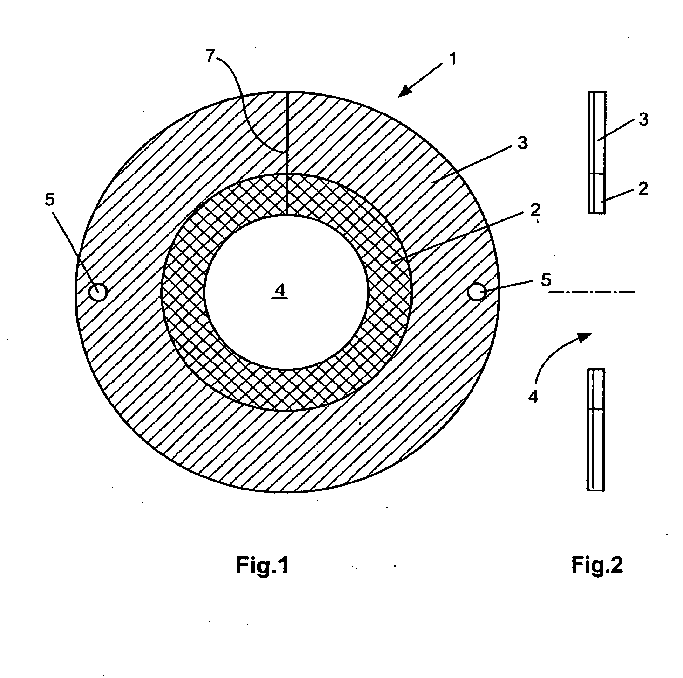

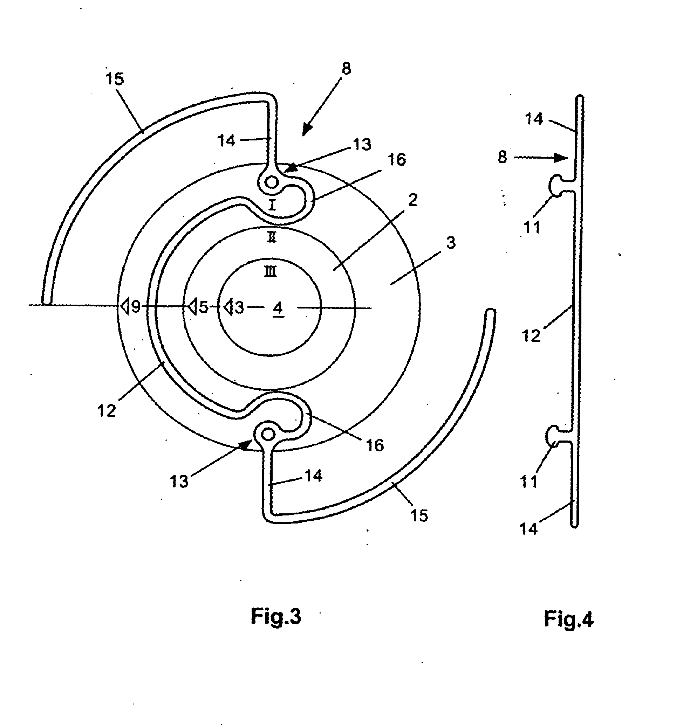

Wie anhand der Fig. 3 bis 6 erläutert wird, kann der in den Fig. 1 und 2 dargestellte Prothesekörper 1 mit unterschiedlichen Trägersystem (Haptiken) verbunden werden, um den Prothesekörper ubiquitär im Bereich des Augenvorderabschnittes einzusetzen. Um den Prothesekörper 1 mit den verschiedenen Trägersystem verbinden zu können, sind am Prothesekörper 1 Öffnungen 5 im Bereich des äußeren Ringbereiches 3 vorgesehen. Im dargestellten Ausführungsbeispiel sind zwei diametral zueinander liegende Öffnungen 5 im äußeren Ringbereich 3 angeordnet. Diese Öffnungen 5 liegen auf einer im wesentlich senkrecht zu einer Faltungslinie 7 des Prothesekörpers verlaufenden Linie. Um diese Faltungslinie 7 kann der Prothesekörper gefaltet werden. Die Faltungslinie 7 kann sich teilweise oder ganz durch den Prothesekörper erstrecken. Insbesondere wenn der Prothesekörpers aus einem relativ harten Material besteht, wird die Faltungslinie 7 von einer linienförmigen Schwachstelle im Material des Prothesekörpers 1 erzeugt. Falls der Prothesekörpers im äußeren Ringbereich 3 aus einem weichen Material besteht und der innere Ringbereich 2 aus einem härteren Material besteht, genügt es, die Faltungslinie 7 im inneren Ringbereich vorzusehen.

In den Fig. 3 bis 6 sind verschiedenen Trägersysteme (Haptiken) dargestellt, mit denen der Prothesekörper im Vorderabschnitt eines Auges implantiert und fixiert werden kann.

In den Fig. 3 und 4 ist ein Trägersystem (Haptik) 8 dargestellt, mit welchem der einen prothetischen Irisersatz darstellende Prothesekörper 1 mit Sulcuspositionierung im Auge fixiert werden kann. Das Trägersystem weist einen etwa halbkreisförmigen Bügel auf, welcher sich im äußeren Ringbereich 3 zwischen diametralen Befestigungsstellen 13 erstreckt. Die Befestigungsstellen 13 werden von den schon erläuterten Öffnungen 5 im Prothesekörper 1 sowie von an das Trägersystem 8 angeformten Steckköpfen 11 gebildet. Die Steckköpfe 11 sind durch die beiden Öffnungen hindurchgesteckt. Hierdurch werden die schon erwähnten Befestigungsstellen 13 gebildet. Die Befestigungsstellen 13 können auch anderweitig gebildet sein, beispielsweise durch Verschweißen des Trägersystems 8 mit dem Material des Prothesekörpers 1. Bevorzugt kommt das erläuterte mechanische Verbindungssystem in den Befestigungsstellen 13 zum Einsatz.

Der halbkreisförmige Bügel 12 bildet für den Prothesekörper 1 eine Stütze und liegt am Prothesekörper 1 im äußeren Ringbereich 3 an, wie es in der Draufsicht der Fig. 3 zu ersehen ist. Von den Befestigungsstellen 13 des Trägersystems erstrecken sich radiale Haptikteile 14 radial nach außen. An den Enden der radialen Haptikteile 13 sind bogenförmige Abdeckteile 15 vorgesehen. Die insbesondere kreisbogenförmig ausgebildeten Haptikteile 15 liegen im implantierten Zustand im Sulcus des Auges. Die bogenförmigen Haptikteile 15 sind vorzugsweise diametral zueinander angeordnet. Es wird hierdurch eine stabile Zentrierung des Prothesekörpers 1 im Auge erreicht. Gewährleistet wird dies durch gleiche Längen der radialen Haptikteile 15, welche mit den gleiche Abstände von der Aperturmitte aufweisenden Befestigungsstellen 13 verbunden sind.

Der etwa halbkreisförmige Bügel 12 kann an seinen Enden mit den Befestigungsstellen 13 verbunden sein. Um jedoch eine vergrößerte Stützfläche zu erreichen, kann der etwa halbkreisförmige Bügel 12 an seinen beiden Enden schleifenförmige Haptikteile 16 aufweisen, welche insbesondere im Bereich der Befestigungsstellen 13 eine vergrößerte Stützwirkung für den Prothesekörper 1 haben. Das jeweilige schleifenförmige Haptikteil 16 ist an der jeweiligen Befestigungsstelle 13 vorbeigeführt und zur Befestigungsstelle 13 bogenförmig zurückgeführt. Wie aus der Draufsicht der Fig. 3 zu ersehen ist, verlaufen die schleifenförmigen Haptikteile 16 radial innerhalb der Befestigungsstellen 13.

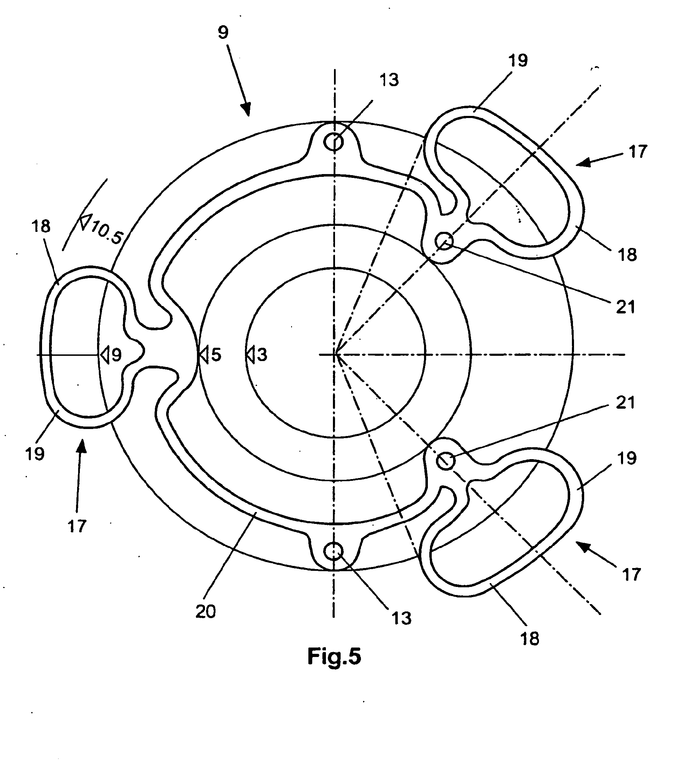

Das Trägersystem 8 ist vorzugsweise einstückig aus einem drahtförmigen Material, welches zumindest an seiner Oberfläche bioverträglich ausgebildet ist, hergestellt und einstückig zu einem Formkörper mit der beschriebenen und in den Fig. 3 und 4 dargestellten Gestalt geformt. Der Durchmesser des Formkörpers kann beispielsweise etwa 2,0 mm bis 4,0 mm betragen. Auch die Trägersysteme der Ausführungsbeispiele der Fig. 5 bis 7 können aus einem derartigen Trägermaterial geformt sein.

Der annährend halbkreisförmige Bügel 12 erstreckt sich beim dargestellten Ausführungsbeispiel der Fig. 3 und 4 etwa über einen Winkelbereich zwischen 140° und 150° und geht dann in die beschriebenen schleifenförmigen Haptikteilen an den Enden über.

Beim Ausführungsbeispiel der Fig. 5 ist das Trägersystem 9 für eine Fixierung des Prothesekörpers 1 am natürlichen Irisgewebe des Auges ausgebildet. Hierzu besitzt das Trägersystem 9 drei Gewebeklammern 17, welche federnd aufeinander zu vorgespannte Zangenteile 18 und 19 aufweisen. Das Irisgewebe wird bei der Fixation zwischen den Enden der aufeinander zu vorgespannten Zangenteilen 18 und 19 eingeklemmt. Die Gewebeklammern 17 sind mit gleichen Winkelabständen voneinander um die Mitte der Apertur 4 des Prothesekörpers 1 angeordnet. Wie beim Ausführungsbeispiel der Fig. 3 und 4 sind Befestigungsstellen 13 im äußeren Ringbereich 3 des Prothesekörpers 1 vorgesehen. Die Befestigungsstellen werden ebenfalls von durch die Öffnungen 5 gesteckten Steckköpfen 11 gebildet. Das Trägersystem 9 besitzt einen etwa 2/3-kreisförmigen Bügel 20, an welchem die Gewebeklammern 17 befestigt sind. Zwei Gewebeklammern befinden sich am Ende des 2/3-kreisförmigen (270°) Bügels 20 und eine Gewebeklammer befindet sich etwa in der Mitte des Bügels 20. Die Befestigungsstellen 13 sind zwischen den jeweiligen an den Bügelenden angeordneten Gewebeklammern und in der Mitte angeordneten Gewebeklammer vorgesehen, wie es aus der Fig. 5 zu ersehen ist. Das Trägersystem 9 der Fig. 5 besteht ebenfalls im wesentlichen aus einem drahtförmigen Material, wobei die Verbindungsstellen zu den Befestigungsstellen 13 und zu den Gewebeklammern 17 abgeflacht sein können. Im Bereich der an den Bügelenden vorgesehenen Gewebeklammern 17 können zusätzliche Befestigungsstellen 21 vorgesehen sein, welche ebenfalls von durch Öffnungen im äußeren Ringbereich 3 des Prothesekörpers 1 hindurchgesteckten Steckköpfen, wie beim Ausführungsbeispiel der Fig. 3 und 4 gebildet sein können.

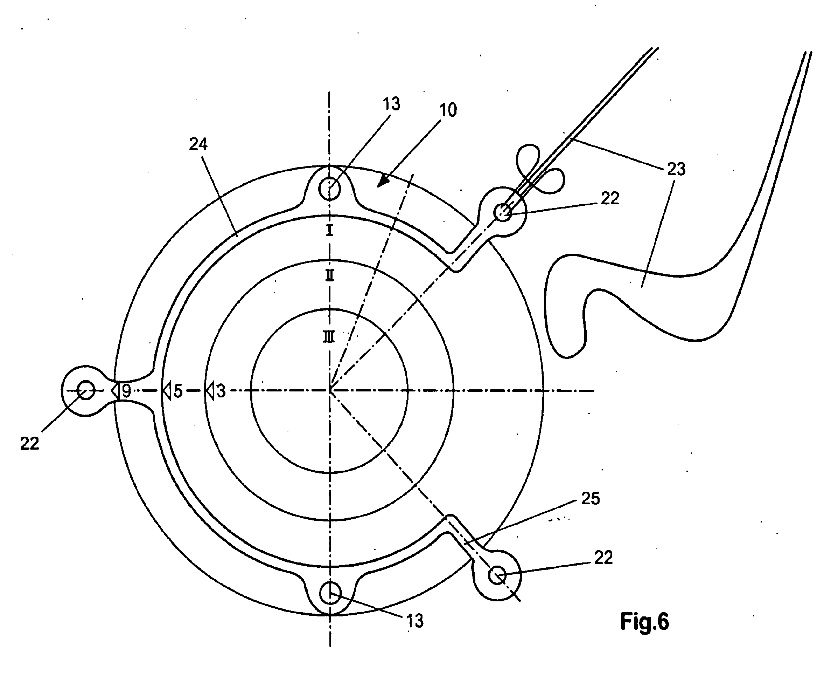

Beim Ausführungsbeispiel der Fig. 6 ist das Trägersystem 10 so ausgebildet, dass eine Sklerafixation des Prothesekörpers 1 ermöglicht wird. Das ebenfalls im wesentlichen aus einem fadenförmigen einstückigen Formkörper gebildeten Trägersystem 10 besitzt einen 2/3-kreisförmigen Bügel 24, in dessen Mitte und an dessen beiden Enden Befestigungsösen 22 vorgesehen sind. Die Befestigungsösen 22 sind um die Mitte der Apertur 4 in gleichen Winkelabständen voneinander angeordnet. Wie beim Ausführungsbeispiel der Fig. 5, befinden sich die Befestigungsstellen 13 zwischen den jeweiligen an den Bügelenden vorgesehenen Befestigungsösen 22 und der in der Mitte angeordneten Befestigungsöse. Der 2/3-kreisförmige Bügel 24 befindet sich, wie beim Ausführungsbeispiel der Fig. 5 der Bügel 20, im äußeren Ringbereich 3. Die Ösen 22 sind an den äußeren Enden von radial sich erstreckenden Haptikteilen 25 vorgesehen. Die radialen Haptikteile 25 bilden die Verbindungen zwischen den Befestigungsösen 22 und dem 2/3-kreisförmigen Bügel 24. Wie aus der Fig. 6 zu ersehen ist, erstreckt sich der Bügel 24 im äußeren Ringbereich 3 etwa über einen Winkelbereich von etwa 270°.

Wie schon erläutert, bilden die im äußeren Ringbereich 3 sich erstreckenden Bügel 12, 20 und 24 der oben beschriebenen Ausführungsbeispiele mechanische Stützen für das weiche Prothesematerial im äußeren Ringbereich und gewährleisten die erforderliche Stabilität des implantierten Prothesekörpers.

Bei den Ausführungsbeispielen der Fig. 5 und 6 liegen die äußeren Begrenzungslinien der Gewebeklammern 17 und der Befestigungsösen 22 auf einem kreisförmigen Umfang mit einem Durchmesser von etwa 10,5 mm. Der äußere Umfang des Prothesekörpers 1 besitzt einen Durchmesser von etwa 9,0 mm. Der Außenumfang des inneren Ringbereiches 2 besitzt bei den dargestellten Ausführungsbeispielen einen Durchmesser von 5,0 mm. Die Apertur 4 besitzt einen Durchmesser von 3,0 mm. Diese in den Figuren dargestellten Abmessungen sind bevorzugte Abmessungen des Prothesekörpers und des jeweiligen Trägersystems.

Mit Hilfe von durch die Befestigungsösen 22 geschlungenen Fäden 23 kann der Prothesekörper 1 beim Ausführungsbeispiel der Fig. 6 an der Augensklera befestigt werden.

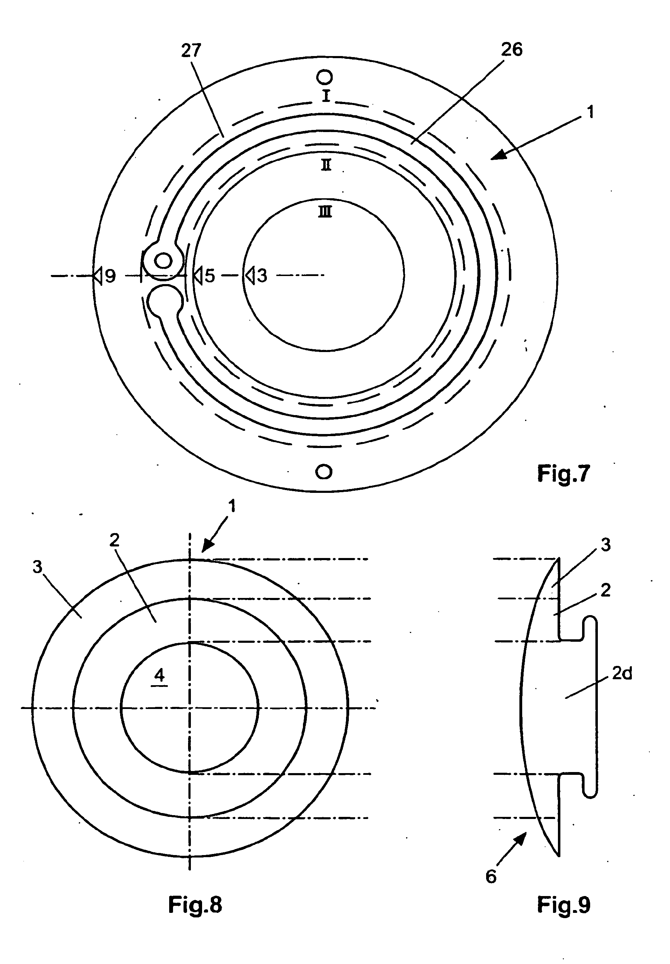

Das in der Fig. 7 dargestellte Ausführungsbeispiel eines prothetischen Irisersatzes ist für eine endokapsuläre Fixation geeignet. Im äußeren Ringbereich 3 des Prothesekörpers 1 ist ein Hohlraum 27, welcher mit strichlierten Begrenzungslinien dargestellt ist, vorgesehen. In diesem Hohlraum 27 befindet sich ein kreisförmiger Spannring 26. Der Spannring 26 besteht aus einem drahtförmigen Material mit bioverträglicher Oberfläche, wie die Trägersysteme 8, 9 und 10 der oben beschriebenen Ausführungsbeispiele. Der Spannring 26 ist um die Mitte der Apertur 4 im Prothesekörper 1 angeordnet. Der Spannring 26 bildet eine stabilisierende Struktur für das insbesondere weiche Material im äußeren Ringbereich 3 des Prothesekörpers 1. Bei der Implantation des Ausführungsbeispiels der Fig. 7 passt sich der scheibenförmige Prothesekörper 1 mit seinem Außendurchmesser von etwa 9,0 mm dem Äquator des Kapselsackes an. Hierdurch wird in Zusammenwirkung mit dem Spannring 26 die Positionierung des Prothesekörpers 1 im Kapselsack des Auges gewährleistet.

Die aus den faden- oder drahtförmigen Formkörpern gebildeten Trägersysteme 8, 9 und 10 und der Spannring 26 bestehen aus einem vorzugsweise härteren Material als das weiche Material des äußeren Ringbereiches 3. Dieses Material besitzt ein federndes Formgedächtnis, d.h. die Trägersysteme 8, 9 und 10 und der Spannring 26 nehmen nach der Implantation ihre ursprüngliche Gestalt, wie sie jeweils in den Figuren dargestellt ist, wieder ein.

Ferner kann der innere Ringbereich 2 an seinem Innenumfang derart ausgebildet sein, dass ein optisches Teil 6 (Fig. 8, 9), insbesondere eine plusbrechende Linse fest eingesetzt werden kann. Der feste Sitz eines optischen Teiles 6 kann ebenfalls durch entsprechende Randgestaltung des Innenumfangrandes des inneren Ringbereiches 2 und des äußeren Umfangsrandes des optischen Teiles 6 erreicht werden. Wenn der innere Ringbereich 2 aus einem harten Material besteht, wird das optische Teil 6 aus einem vorzugsweise elastischen Material, insbesondere Silikonkautschuk hergestellt. Das optische Teil 6 und/oder der innere Ringbereich 2 können als Steckprothetik ausgebildet sein.

Der innere Ringbereich 2 kann auch als Träger für eine andersartige Steckprothetik, beispielsweise eine Occlusionsprothetik, welche die Apertur 4 abdeckt, ausgebildet sein.

Die in den Fig. 8 und 9 dargestellte Steckoptik 6 kann aus Silikonmaterial gebildet sein, welches gegebenenfalls an der Oberfläche hydrophilisiert ist. Es ist jedoch auch möglich, ein anderes geeignetes Kunststoffmaterial, welches zumindest an der Oberfläche biokompatibel gestaltet ist, zu verwenden. Wie in den Fig. 8 und 9 dargestellt ist, können in das Material der Steckoptik 6 die beiden ringförmigen Bereiche 2 und 3 vorgesehen sein. Wie bei den oben beschriebenen Ausführungsbeispielen bildet der innere Ringbereich 2 einen phototrop ausgebildeten Ringbereich und der äußere Ringbereich 2 ist lichtundurchlässig ausgebildet. Vorzugsweise beträgt der Außendurchmesser der Steckoptik 6 bei einer derartigen Ausbildung 7,0 mm. Dies ist auch der Außendurchmesser des lichtundurchlässigen äußeren Ringbereiches 3. Der Außendurchmesser des inneren phototropen Ringbereiches 2 beträgt 5,0 mm und die Apertur 4 hat einen Durchmesser von 3,0 mm. Die Apertur 4 besteht beim dargestellten Ausführungsbeispiel aus lichtdurchlässigen Material.

An der Rückseite der Steckoptik 6 ist ein Steckkopf 28 vorgesehen, welcher, wie oben schon erläutert, durch die Öffnung des Prothesekörpers 1, welcher, wie im Zusammenhang der oben beschriebenen Ausführungsbeispiele im Auge fixiert werden kann, hindurchgesteckt wird.

Die Steckoptik 6 kann jedoch auch so ausgebildet sein, dass um ihre lichtdurchlässige Apertur 4 nur der phototrope Ringbereich 2 angeordnet ist. In diesem Fall beträgt der Außendurchmesser der Steckoptik 6 5,0 mm. Die Apertur hat einen Durchmesser von 3,0 mm.

Es ist auch möglich, bei den Ausführungsbeispielen der Fig. 1 bis 7 eine Steckoptik 6 zu verwenden, welche vollständig aus lichtdurchlässigem Material besteht.

Zur Unterstützung der Faltbarkeit des Prothesekörpers 1 kann vorzugsweise im harten, inneren Ringbereich 2 eine Trenn- oder Schwachstelle 7, welche sich auch im äußeren Ringbereich 3 fortsetzen kann, vorgesehen sein. Es können auch mehrere radial sich erstreckende Trenn- oder Schwachstellen im inneren Ringbereich 2 oder Prothesekörper 1 vorgesehen sein. Durch die Schwachstellen 7 wird eine Faltbarkeit auch des inneren Ringbereiches 2 erreicht.

### [Bezugszeichenliste]

- 1: Prothesekörper
- 2: innerer Ringbereich
- 3: äußerer Ringbereich
- 4: Apertur
- 5: Befestigungsstellen (Öffnungen)
- 6: Steckort
- 7: Faltlinie
- 8,9,10: Trägersysteme (Haptiken)
- 11: Steckköpfe
- 12: etwa halbkreisförmiger Bügel
- 13: Befestigungsstelle
- 14: radiale Haptikteile
- 15: bogenförmige Haptikteile
- 16: schleifenförmige Haptikteile
- 17: Gewebeklammern
- 18,19: Hängeteile
- 20: 2/3-kreisförmiger Bügel
- 21: zusätzliche Befestigungsstelle
- 22: Befestigungsöse
- 23: Faden
- 24: 2/3-kreisförmiger Bügel
- 25: radiale Haptikteile
- 26: Spannring
- 27: Hohlraum
- 28: Steckkopf

## Patentansprüche

1. Künstlicher, in ein Auge implantierbarer Irisersatz aus einem augenverträglichen Material mit einem eine mittlere Apertur aufweisenden Prothesekörper (1), der einen inneren, die Apertur (4) umfassenden phototrop ausgebildeten Ringbereich (2) und einen den inneren Ringbereich (2) umfassenden, äußeren, im wesentlichen lichtundurchlässig ausgebildeten Ringbereich (3) aufweist, und mit einer Haptik,
**dadurch gekennzeichnet, dass** am Prothesekörper (1) unterschiedliche Trägersysteme (8, 9, 10), welche für Implantationen im Vorderabschnitt des Auges angepasst sind, befestigbar sind, wobei die Trägersysteme (8, 9, 10) Steckköpfe (11) aufweisen, die in Öffnungen (5), welche im äußeren Ringbereich (3) des Prothesekörpers (1) vorgesehen sind, einsteckbar sind.

2. Irisersatz nach Ansprüch 1,
**dadurch gekennzeichnet, dass** der äußere lichtundurchlässige Ringbereich (3) ein Irismuster aufweist.

3. Irisersatz nach Ansprüche 1 oder 2,
**dadurch gekennzeichnet, dass** der Prothesekörper (1) ganz oder zumindest im äußeren Ringbereich (3) aus einem weichen Material besteht.

4. Irisersatz nach einem der Ansprüche 1 bis 3,
**dadurch gekennzeichnet, dass** der innere phototrop ausgebildete Ringbereich (2) aus einem härteren Material besteht als der äußere Ringbereich (3).

5. Irisersatz nach einem der Ansprüche 1 bis 4,
**dadurch gekennzeichnet, dass** in den äußeren Ringbereich (3) ein den phototropen Ringbereich (2) umgebender Stabiiisierungsring (26) aus einem härteren Material als der äußere Ringbereich (3) eingelagert ist.

6. Irisersatz nach einem der Ansprüche 1 bis 5,
**dadurch gekennzeichnet, dass** der innere Ringbereich (2) faltbar oder klappbar ausgebildet ist.

7. Irisersatz nach einem der Ansprüche 1 bis 6,
**dadurch gekennzeichnet, dass** in die Apertur (4) eine Steckprothese einsetzbar ist.

8. Irisersatz nach Anspruch 7,
**dadurch gekennzeichnet, dass** die Steckprothese als Steckoptik, insbesondere Linse oder als Occlusionsprothetik ausgebildet ist.

9. Irisersatz nach Anspruch 1,
**dadurch gekennzeichnet, dass** die Trägersysteme (8, 9, 10) Bügel (12, 20, 24) aufweisen, welche im äußeren Ringbereich (3) am Prothesekörper (1) anliegen.

10. Irisersatz nach Anspruch 1,
**dadurch gekennzeichnet, dass** für eine Sulcusfixation am Trägersystem (8) bogenförmige im Sulcus des Auges platzierbare Haptikteile (15) vorgesehen sind die über radial verlaufende Haptikteile (14) mit dem Bügel (12) und/oder den Befestigungsstellen (13) verbunden sind.

11. Irisersatz nach Anspruch 1,
**dadurch gekennzeichnet, dass** für eine Irisfixation am Trägersystem (9) Gewebeklammern (17) vorgesehen sind, welche am Irisgewebe durch federnden Zangeneingriff fixierbar sind.

12. Irisersatz nach Anspruch 11,
**dadurch gekennzeichnet, dass** drei in gleichen Winkelabständen voneinander angeordnete Gewebeklammem (17) am Trägersystem (9) vorgesehen sind.

13. Irisersatz nach Anspruch 1,
**dadurch gekennzeichnet, dass** für eine Sklerafixation am Trägersystem (10) drei Befestigungsösen angeordnet sind, durch die an der Sklera fixierbare Fäden (23) geschlungen werden können.

14. Irisersatz nach Anspruch 8
**dadurch gekennzeichnet, dass** die Steckoptik (6) einen um eine aus lichtdurchlässigem Material bestehende Apertur (4) angeordneten phototropen Ringbereich (2) aufweist.

15. Irisersatz nach Anspruch 14,
**dadurch gekennzeichnet, dass** um den phototropen Ringbereich (2) ein äußerer lichtundurchlässiger Ringbereich (3) angeordnet ist.

16. Irisersatz nach einem der Ansprüche 8, 14, 15,
**dadurch gekennzeichnet, dass** die Steckoptik (6) insgesamt aus lichtdurchlässigem Material besteht.

17. Irisersatz nach einem der Ansprüche 1 bis 16,
**dadurch gekennzeichnet, dass** der Prothesekörper (1) als Intraokularlinse ausgebildet ist, die um die lichtdurchlässige Apertur (4) in der Linsenmitte den phototropen Ringbereich (2) aufweist.

18. Irisersatz nach Anspruch 17,
**dadurch gekennzeichnet, dass** um den phototropen Ringbereich (2) der lichtundurchlässige äußere Ringbereich (3) angeordnet ist.

## Claims

1. An artificial iris replacement, implantable into an eye, made of an eye-compatible material, and comprising a prosthesis body (1) with a central aperture, having an inner phototropic ring area (2) surrounding the aperture (4), and an outer ring area (3), which is essentially opaque, surrounding the inner ring area (2), and fixtures,
**characterized in that** various supporting systems (8, 9, 10) adapted for implantation within the anterior section of the eye may be attached to the prosthesis body (1), said supporting systems (8, 9, 10) having connecting pins for insertion into openings (5) provided in the outer ring area (3) of the prosthesis body (1).

2. An iris replacement according to claim 1,
**characterized in that** the outer, opaque, ring area (3) has an iris pattern.

3. An iris replacement according to claims 1 or 2,
**characterized in that** the prosthesis body (1) is entirely, or at least in the outer ring area (3), made of a soft material.

4. An iris replacement according to any of claims 1 to 3,
**characterized in that** the inner, phototropic, ring area (2) is made of a more rigid material than the outer ring area (3).

5. An iris replacement according to any of claims 1 to 4,
**characterized in that** a stabilizing ring (26) made of a more rigid material than the outer ring area (3), and surrounding the phototropic ring area (2), is incorporated in the outer ring area (3).

6. An iris replacement according to any of claims 1 to 5,
**characterized in that** the inner ring area (2) is foldable or collapsible.

7. An iris replacement according to any of claims 1 to 6,
**characterized in that** a plug-in prosthesis can be inserted into the aperture (4).

8. An iris replacement according to claim 7,
**characterized in that** the plug-in prosthesis is a plug-in optical device, in particular a lens or an occlusion prosthesis.

9. An iris replacement according to claim 1,
**characterized in that** the supporting systems (8, 9, 10) comprise clips (12, 20, 24) sitting close to the prosthesis body (1) at the outer ring area (3).

10. An iris replacement according to claim 1,
**characterized in that** for the purpose of sulcus fixation there are provided on the supporting system arc-shaped fastening members (15) to be placed in the sulcus of the eye and which are connected to the clip (12) and/or the attachment points (13) via radially arranged fastening members (14).

11. An iris replacement according to claim 1,
**characterized in that** for the purpose of iris fixation there are provided tissue clips (17) on the supporting system that can be fastened to the iris tissue by a resilient tongs grip.

12. An iris replacement according to claim 11,
**characterized in that** there are provided on the supporting system (9) three tissue clips (17) spaced from one another by the same angular distance.

13. An iris replacement according to claim 1,
**characterized in that** for the purpose of scleral fixation there are provided on the supporting system (10) three fixing eyelets through which threads attachable to the sclera can be wound.

14. An iris replacement according to claim 8,
**characterized in that** the plug-in optics (6) comprises a phototropic ring area (2) placed around an aperture (4) made of a transparent material.

15. An iris replacement according to claim 14,
**characterized in that** an outer opaque ring area (3) is provided around the phototropic ring area (2).

16. An iris replacement according to any of claims 8, 14, 15,
**characterized in that** the whole of the plug-in optics is made of a transparent material.

17. An iris replacement according to any of claims 1 to 16,
**characterized in that** the prosthesis body (1) is formed as an intraocular lens comprising the phototropic ring area (2) around the light transmitting aperture (4) in the middle of the lens.

18. An iris replacement according to claim 17,
**characterized in that** the opaque outer ring area (3) is provided around the phototropic ring area (2).

## Revendications

1. Iris artificiel, implantable dans un oeil, en une matière compatible avec l'oeil, avec un corps de prothèse (1) pourvu d'une ouverture centrale, qui présente une zone annulaire intérieure (2) de réalisation phototropique, entourant l'ouverture (4), et une zone annulaire extérieure (3) de réalisation essentiellement opaque qui entoure la zone annulaire intérieure (2), et avec une haptique,
**caractérisé en ce que** différents systèmes supports (8, 9, 10), adaptés pour des implantations dans la section avant de l'oeil, peuvent être fixés sur le corps de prothèse (1), les systèmes supports (8, 9, 10) présentant des têtes d'emboîtement (11) qui peuvent être emmanchées dans des ouvertures (5) prévues dans la zone annulaire extérieure (3) du corps de prothèse (1).

2. Iris artificiel suivant la revendication 1, **caractérisé en ce que** la zone annulaire extérieure opaque (3) présente un modèle d'iris.

3. Iris artificiel suivant l'une des revendications 1 et 2, **caractérisé en ce que** le corps de prothèse (1) se compose totalement, ou au moins dans la zone annulaire extérieure (3), d'une matière souple.

4. Iris artificiel suivant l'une des revendications 1 à 3, **caractérisé en ce que** la zone annulaire intérieure (2), de réalisation phototropique, se compose d'une matière plus rigide que celle de la zone extérieure annulaire (3).

5. Iris artificiel suivant l'une des revendications 1 à 4, **caractérisé en ce qu'**une bague de stabilisation (26) en une matière plus rigide que celle de la zone annulaire extérieure (3) et qui entoure la zone annulaire phototropique (2), est intercalée dans la zone annulaire extérieure (3).

6. Iris artificiel suivant l'une des revendications 1 à 5, **caractérisé en ce que** la zone annulaire intérieure (2) a une réalisation pliable ou rabattable.

7. Iris artificiel suivant l'une des revendications 1 à 6, **caractérisé en ce qu'**une prothèse emboîtable peut être mise en place dans l'ouverture (4).

8. Iris artificiel suivant la revendication 7, **caractérisé en ce que** la prothèse emboîtable est réalisée sous forme d'optique enfichable, en particulier de lentille ou sous forme de prothétique occlusive.

9. Iris artificiel suivant la revendication 1, **caractérisé en ce que** les systèmes supports (8 , 9, 10) présentent des arceaux (12, 20, 24), qui s'appliquent sur le corps de prothèse (1) dans la zone annulaire extérieure (3).

10. Iris artificiel suivant la revendication 1, **caractérisé en ce que** des éléments haptiques (14) en forme d'arc, pouvant être placés dans le sulcus de l'oeil, sont prévus pour une fixation du sulcus sur le système support (8), lesquels éléments haptiques sont assemblés, par l'intermédiaire d'éléments haptiques radiaux (14), avec l'arceau (12) et/ou les points de fixation (13).

11. Iris artificiel suivant la revendication 1, **caractérisé en ce que** des agrafes en tissu (17) sont prévues pour une fixation de l'iris sur le système support (9), lesquelles agrafes peuvent être fixées sur le tissu iridien par prise de pince élastique.

12. Iris artificiel suivant la revendication 11, **caractérisé en ce que** trois agrafes en tissu (17), disposées à des intervalles angulaires égaux les unes des autres, sont prévues sur le système support (9).

13. Iris artificiel suivant la revendication 1, **caractérisé en ce que** trois oeillets de fixation sont disposés pour une fixation de la sclérotique sur le système support (10), oeillets au travers desquels peuvent être enlacés des fils (23) fixables sur la sclérotique.

14. Iris artificiel suivant la revendication 8**, caractérisé en ce que** l'optique enfichable (6) présente une zone annulaire (2) phototropique disposée autour d'une ouverture (4) composée d'une matière transparente.

15. Iris artificiel suivant la revendication 14, **caractérisé en ce qu'**une zone annulaire (3) extérieure opaque est disposée autour de la zone annulaire (2) phototropique.

16. Iris artificiel suivant l'une des revendications 8, 14, 15, **caractérisé en ce que** l'optique enfichable (6) se compose dans l'ensemble d'une matière transparente.

17. Iris artificiel suivant l'une des revendications 1 à 16, **caractérisé en ce que** le corps de prothèse (1) est réalisé sous forme de lentille intraoculaire, qui présente la zone annulaire (2) phototropique autour de l'ouverture (4) transparente au centre de la lentille.

18. Iris artificiel suivant la revendication 17, **caractérisé en ce que** la zone annulaire extérieure (3) opaque est disposée autour de la zone annulaire (2) phototropique.
